# EUROPÄISCHE PATENTSCHRIFT

(11) **EP 1 952 773 B1**
(45) Veröffentlichungstag und Bekanntmachung des Hinweises auf die Patenterteilung: **04.05.2011**
(21) Anmeldenummer: 08001608.2
(22) Anmeldetag: 29.01.2008
(51) Int. Cl.: A61B 17/32, A61B 17/28

(54) **Medizinisches Instrument zum Schneiden von Gewebe**
Medical instrument for cutting tissue
Instrument médical destiné à couper des tissus

(30) Priorität: 31.01.2007 DE 102007006276
(43) Veröffentlichungstag der Anmeldung: 06.08.2008
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Heuckeroth, Volker

(56) Entgegenhaltungen:
- WO-A2-97/45054
- US-A- 5 492 527
- US-A- 5 665 101
- US-A1- 2004 147 909
- US-A1- 2005 159 767

## Beschreibung

Die Erfindung betrifft ein medizinisches Instrument zum Schneiden von Gewebe, mit einem rohrförmigen Außenschaft, der im Bereich seines distalen Endes zumindest ein Fenster aufweist, mit einem rohrförmigen, um eine Längsachse rotierbaren, in dem Außenschaft aufgenommenen Innenschaft, der an seinem distalen Ende ein im Bereich des zumindest einen Fensters des Außenschafts angeordnetes und mit zumindest einem Schneidelement versehenes Fenster aufweist.

Ein derartiges medizinisches Instrument ist aus US 5,492,527 bekannt.

Solche Instrumente, die auch als Rotationsschneidinstrumente oder Shaver bekannt sind, werden bspw. in der minimal-invasiven Chirurgie zum Abtrennen von Gewebe im menschlichen oder tierischen Körper verwendet.

Das bekannte medizinische Instrument weist einen Handgriff auf, der distalseitig drehfest mit einem rohrförmigen Außenschaft verbunden ist. Der Außenschaft weist an seinem distalen Ende ein Fenster auf. Im Außenschaft ist ein rohrförmiger Innenschaft aufgenommen, der an seinem distalen Ende im Bereich des Fensters des Außenschafts ebenfalls ein Fenster mit zumindest einem Schneidelement aufweist. Der Innenschaft ist proximalseitig mit Antriebsmitteln, bspw. mit einem internen oder externen elektrischen Motor, verbindbar, um den Innenschaft relativ zum Außenschaft um eine Längsachse in Rotation zu versetzen.

Um Gewebe abzutragen, wirkt das Schneidelement während eines Umlaufs des Innenschafts mit dem Fenster des Außenschafts derart zusammen, dass das Schneidelement an jenem vorbei läuft und somit seitlich des Außenschafts befindliches abtrennt. Das abgetragene Gewebe wird durch das Fenster über den Innenschaft, an dem eine Saugleitung angeschlossen ist, nach proximal abgesaugt.

Die anfängliche Stellung des Außen- und Innenschafts kann durch den durchzuführenden operativen Eingriff bedingt sein, wobei die vorgegebene Stellung beider Bauteile bspw. dadurch definiert ist, dass die Fenster des Außen- und Innenschafts in einer bestimmten Weise relativ zueinander angeordnet sind. Dabei kann es erwünscht sein, dass der Außenschaft und der Innenschaft eine solche Drehstellung relativ zueinander einnehmen, dass das Fenster des Außenschafts durch das distale Ende des Innenschafts verschlossen ist, wodurch eine Trennung des Innenschafts von der äußeren Umgebung bewirkt wird. Dies ist insbesondere im Rahmen von Eingriffen beim Einführen des distalen Endes des Außenschafts in das Operationsgebiet mit bereits angeschlossener und aktivierter Saugleitung erforderlich, um ein Ansaugen von unbeteiligtem Gewebe oder Flüssigkeit zu vermeiden, wodurch zum einen eine Schädigung des unbeteiligten Gewebes und zum anderen ein Verstopfen des distalen Endes des Schafts vermieden wird. In anderen Fällen kann es gewünscht sein, dass der Innenschaft relativ zum Außenschaft eine Drehstellung einnimmt, in der das Fenster des Außenschafts maximal geöffnet ist. Eine derartige Stellung des Innenschafts relativ zum Außenschaft ist bspw. für Eingriffe im Bereich der Hals-Nasen-Ohren-Heilkunde vorteilhaft, bei denen das distale Ende des Außenschafts nicht tief in das Operationsgebiet eingebracht und schon beim Einführen Gewebe oder Flüssigkeit abgesaugt wird, um eine bessere Sichtkontrolle für den Arzt zu erreichen.

Das aus der eingangs genannten US 5,492,527 bekannte medizinische Instrument weist ferner eine zwischen dem Außenschaft und dem Handgriff vorgesehene Haltevorrichtung auf, mit der das Fenster des Außenschafts relativ zum Handgriff drehfest in einer definierten Position angeordnet werden kann. Die Haltevorrichtung ist als zwei ineinander greifende Zahnkränze mit flachen Zähnen ausgebildet, die durch eine axiale Bewegung einer die Zahnkränze aufnehmenden Hülse gegeneinander verschoben werden können. Hierdurch kann der Außenschaft relativ zum Handgriff in 15°-Schritten um die Längsachse des medizinischen Instruments gedreht werden, während der Innenschaft in seiner anfänglichen Stellung verbleibt. Im Betriebszustand des medizinischen Instruments rotiert der Innenschaft um die Längsachse, während der Außenschaft in der zuvor eingestellten Position gehalten wird.

Ein Nachteil dieses medizinischen Instruments ist es, dass die Haltevorrichtung den Außenschaft zwar relativ zum Handgriff in einer gewünschten Stellung positioniert, nicht aber den Außenschaft und den Innenschaft relativ zueinander in einer vorbestimmten Drehstellung hält. Der Innenschaft ist frei drehbar im Außenschaft aufgenommen, so dass hierdurch die anfängliche Drehstellung beider Bauteile, insbesondere die relative Lage beider Fenster zueinander, durch eine ungewollte Verdrehung des Innenschafts verändert werden kann. Diese leicht veränderbare Relativanordnung des Innen- und des Außenschafts kann insbesondere beim Einführen des Instruments in den Körper mit aktivierter Saugleitung zu dem oben beschriebenen unerwünschten Ansaugen von unbeteiligtem Gewebe oder Flüssigkeit führen.

Es ist ferner nachteilig, dass der Außenschaft eine ungewollte Drehbewegung ausführen kann, da die Zahnkränze derart flach ausgebildet sind, dass sie auch ohne axiales Verschieben der Hülse leicht gegeneinander verschoben werden können. Die relative Stellung des Außenschafts zum Innenschaft kann folglich durch eine kleine Unachtsamkeit des Arztes, bspw. durch eine ungewollte, manuell erzwungene Drehung des Außenschafts, verändert werden, so dass sich der Außenschaft relativ zum Handgriff und auch relativ zum Innenschaft verdreht und sich die vorgegebene Stellung der jeweiligen Bauteile ändert. Somit erfordert das Bedienen des medizinischen Instruments eine erhöhte Achtsamkeit und Vorsicht seitens des Arztes.

Ein weiterer Nachteil ist, dass die Haltevorrichtung konstruktiv sehr aufwendig ausgebildet ist und folglich hohe Herstellungskosten für das medizinische Instrument bedingt.

Es ist daher eine Aufgabe der vorliegenden Erfindung, das medizinische Instrument der eingangs genannten Art derart zu verbessern, dass mit einer konstruktiv einfachen Maßnahme gewährleistet ist, dass der Innenschaft beim Einführen des Schafts in das Operationsgebiet relativ zum Außenschaft eine vorbestimmte Drehstellung beibehält.

Erfindungsgemäß wird die Aufgabe hinsichtlich des eingangs genannten medizinischen Instruments durch ein Fixierelement zum drehfesten Halten des Innenschafts relativ zum Außenschaft gelöst, wobei das Fixierelement durch eine Rotationsbewegung des Innenschafts irreversibel lösbar ist.

Das erfindungsgemäße medizinische Instrument zum Schneiden von Gewebe weist einen rohrförmigen Außenschaft auf, in dem ein rohrförmiger, um eine Längsachse rotierbarer Innenschaft aufgenommen ist. Der Außenschaft und der Innenschaft weisen jeweils an ihrem distalen Ende ein Fenster auf. Das Fenster des Innenschafts ist im Bereich des Fensters des Außenschafts angeordnet und weist zumindest eine Schneidkante auf. Das medizinische Instrument weist ferner ein Fixierelement auf, das den Innenschaft relativ zum Außenschaft drehfest in einer anfänglich vorgegebenen Stellung hält und das durch eine Rotationsbewegung des Innenschafts irreversibel lösbar ist. Somit weist das erfindungsgemäße medizinische Instrument vorteilhafterweise einen Mechanismus auf, mit dem der Innenschaft relativ zum Außenschaft so lange drehfest gehalten wird, bis der Innenschaft bspw. durch Einschalten des Drehantriebs in Drehung versetzt wird, wodurch die vorgegebene Position der Fenster des Außen- und Innenschafts beim Einführen des Schafts in das Operationsgebiet sicher beibehalten wird. Ist die vorgegebene Drehstellung derart, dass das Außenfenster geschlossen ist, so bleibt beim Einführen des Schafts in das Operationsgebiet das Außenfenster sicher geschlossen, so dass bei bereits anliegendem Saugstrom dieser nicht durch das Außenfenster auf das umliegende Gewebe wirkt. Daher gestaltet sich die Handhabung des medizinischen Instruments besonders benutzerfreundlich, da der Arzt nicht auf eine ungewollte Veränderung einer vorgegebenen Stellung des Außen- und Innenschafts achten muss.

Auch das Lösen der Fixierung zwischen Innenschaft und Außenschaft ist vorteilhaft einfach, da zum Lösen lediglich der Innenschaft durch Einschalten des Antriebs in Drehung versetzt werden muss, ohne dass zum Lösen ein Eingreifen des Bedieners erforderlich ist.

Ein weiterer Vorteil des medizinischen Instruments ergibt sich dadurch, dass das Fixierelement bei Inbetriebnahme des Instruments, d.h. bei einer Rotation des Innenschafts relativ zum Außenschaft, irreversibel gelöst wird. Hierdurch ist es nämlich schon vor der Operation durch den Arzt überprüfbar, ob das medizinische Instrument beschädigt ist und gegen ein neues mit noch nicht gelöstem Fixiermechanismus ausgetauscht werden muss.

Das erfindungsgemäße medizinische Instrument ist aufgrund seines irreversibel lösbaren Fixierelements insbesondere als Einweginstrument geeignet. Das Fixierelement kann aus einem brechbaren Material, bspw. Kunststoff, gefertigt sein, das reproduzierbar in einem definierten Bereich, d. h. an einer Sollbruchstelle oder entlang einer Sollbruchlinie, zerbricht. Es ist ebenfalls möglich, dass das Fixierelement aus elastischem, reißfähigem Material, bspw. Gummi, ausgebildet ist, das infolge einer Rotationsbewegung des Innenschafts reißt.

In einer bevorzugten Ausgestaltung der Erfindung verbindet das Fixierelement ein mit dem Außenschaft drehfest verbundenes Kupplungselement zum Verbinden des Außenschafts mit einem Handgriff mit einem mit dem Innenschaft drehfest verbundenen Rotationsübertragungselement zum Verbinden des Innenschafts mit Antriebsmitteln.

Das medizinische Instrument weist ferner ein Kupplungselement und ein Rotationsübertragungselement auf, das mit dem Außenschaft bzw. dem Innenschaft drehfest verbunden ist. Das Fixierelement bewirkt eine irreversibel lösbare Verbindung dieser beiden Bauteile. Somit ist das Fixierelement vorteilhafterweise nicht direkt am Innen- und Außenschaft angeordnet, so dass eine Beschädigung dieser beiden Bauteile aufgrund eines Brechens oder Reißens des Fixierelements vermieden wird, die sich auch auf die Relativrotationsbewegung des Innen- und Außenschafts und folglich auf die Schneidwirkung des medizinischen Instruments nachteilig auswirken kann.

In einer weiteren bevorzugten Ausgestaltung der Erfindung ist das Fixierelement an einer äußeren Oberfläche des Kupplungselements und des Rotationsübertragungselements angeordnet.

Diese Maßnahme hat den Vorteil, dass jede beliebige Anordnung des Innenschafts relativ zum Außenschaft ermöglicht wird, da das Fixierelement an jeder Position auf der Oberfläche des Kupplungselements und des Rotationsübertragungselements vorgesehen sein kann. Ferner kann das Fixierelement beim Fertigungsprozess vorteilhafterweise nachträglich auf die äußere Oberfläche beider Bauteile aufgebracht werden, wodurch die Herstellung des medizinischen Instruments flexibel an die jeweiligen Operationserfordernisse angepasst werden kann. Zudem kann der Arzt vor Gebrauch des medizinischen Instruments optisch auf einfache Weise überprüfen, ob das Fixierelement einen zum Gebrauch geeigneten Sollzustand oder eine Beschädigung aufweist.

In einer weiteren bevorzugten Ausgestaltung der Erfindung ist das Fixierelement zumindest teilweise umfänglich am Kupplungselement und/oder Rotationsübertragungselement angeordnet.

Diese Maßnahme hat den Vorteil, dass im Gegensatz zu einer Anordnung des Fixierelements an lokalen Bereichen des Kupplungs- und/oder Rotationsübertragungselements eine besonders stabile, drehfeste Verbindung des Außen- und Innenschafts bewirkt wird.

In einer weiteren bevorzugten Ausgestaltung der Erfindung sind das Kupplungselement und das Fixierelement einstückig ausgebildet.

In einer weiteren bevorzugten Ausgestaltung der Erfindung sind das Rotationsübertragungselement und das Fixierelement einstückig ausgebildet.

Diese Maßnahmen haben den Vorteil, dass die jeweiligen Bauteile in einem einzigen Produktionsschritt hergestellt werden können. Hierdurch ergibt sich eine besonders schnelle und gleichzeitig kostengünstige Fertigung des medizinischen Instruments. Insbesondere können das Kupplungselement, das Fixierelement und das Rotationsübertragungselement einstückig ausgebildet sein.

In einer weiteren bevorzugten Ausgestaltung der Erfindung weist das Fixierelement zumindest einen länglichen Steg auf, der durch die Rotationsbewegung des Innenschafts an zumindest einer Sollbruchstelle bricht.

Diese Maßnahme ermöglicht vorteilhafterweise eine konstruktiv besonders einfache und kostengünstige Ausgestaltung des Fixierelements, das eine stabile, drehfeste Verbindung des Außen- und Innenschafts gewährleistet und infolge von bei der Rotationsbewegung des Innenschafts auftretenden Torsions- und Scherkräfte in einem definierten Bereich brechen kann. Insbesondere ist ein Brechen des Fixierelements an einer zuvor bestimmbaren Sollbruchstelle vorteilhaft, da hierbei ein Zersplittern des Fixierelements in kleinste Teile vermieden wird, die innerhalb des medizinischen Instruments verbleiben und die Relativrotation des Außen- und Innenschafts behindern können.

In einer weiteren bevorzugten Ausgestaltung der Erfindung ist der zumindest eine Steg entlang der Längsachse angeordnet.

Diese Maßnahme hat den Vorteil, dass durch die Anordnung des Stegs parallel zur Längsachse die Verbindung des Kupplungselements mit dem Rotationsübertragungselement gleichermaßen stabil gegen kleine Drehmomente in beiden Rotationsrichtungen ist, so dass ein ungewolltes Lösen des Stegs verhindert wird.

In einer alternativen bevorzugten Ausgestaltung der Erfindung weist das Fixierelement einen Ring auf, der durch die Rotationsbewegung des Innenschafts entlang einer Sollbruchlinie bricht.

Diese Maßnahme stellt vorteilhafterweise eine weitere kostengünstige, einfache Bauart des Fixierelements dar, die ebenfalls aufgrund einer vollumfänglichen Anordnung des Rings eine besonders stabile Verbindung des Kupplungselements mit dem Rotationsübertragungselement gewährleistet, so dass ein ungewolltes Verdrehen des Innenschafts relativ zum Außenschaft verhindert wird. Der Ring kann dadurch irreversibel gelöst werden, dass er entlang einer Sollbruchlinie bricht.

In einer weiteren bevorzugten Ausgestaltung der Erfindung weist das medizinische Instrument einen Positionsindikator zum Ausrichten des Innenschafts relativ zum Außenschaft in der vorgegebenen Stellung auf, wobei der Positionsindikator in einer Flucht mit dem Fixierelement angeordnet ist.

Der Positionsindikator dient zum Ausrichten des Rotationsübertragungselements und des Kupplungselements, d.h. des Innenschafts und des Außenschafts, relativ zueinander während der Fertigung des medizinischen Instruments. Die Anordnung der jeweiligen Bauteile kann vorteilhafterweise nachträglich durch den Arzt überprüft werden, so dass bereits vor Durchführen des Eingriffs eine abweichende Anordnung der Bauteile erkannt wird und das medizinische Instrument gegen ein anderes ausgetauscht werden kann.

In einer weiteren bevorzugten Ausgestaltung der Erfindung ist der Positionsindikator an der äußeren Oberfläche des Kupplungselements angeordnet.

Diese Maßnahme ermöglicht vorteilhafterweise eine für den Arzt sichtbare Anordnungsmöglichkeit des Positionsindikators im Vergleich zu einer Anordnung bspw. auf einer Oberfläche des Rotationsübertragungselements, da das Kupplungselement frei zugänglich ist und nicht wie das Rotationsübertragungselement durch ein anderes Bauteil verdeckt werden kann.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit der beiliegenden Zeichnung näher beschrieben und erläutert. Es zeigen:
- Fig. 1: ein medizinisches Instrument zum Schneiden von Gewebe gemäß der vorliegenden Erfindung im Querschnitt;
- Fig. 2: eine perspektivische Darstellung des medizinischen Instruments in Fig.1 in Explosionsansicht;
- Fig. 3: eine weitere perspektivische Darstellung des medizinischen Instruments in Fig. 1, das ein erstes Ausführungsbeispiel eines Fixierelements aufweist;
- Fig. 4: eine weitere perspektivische Darstellung des medizinischen Instruments in Fig. 3, wobei das Fixierelement irreversibel gelöst ist;
- Fig. 5: eine weitere perspektivische Darstellung des medizinischen Instruments in Fig. 1, das ein zweites Ausführungsbeispiel des Fixierelements aufweist; und
- Fig. 6: eine weitere perspektivische Darstellung des medizinischen Instruments in Fig. 5, wobei das Fixierelement irreversibel gelöst ist;

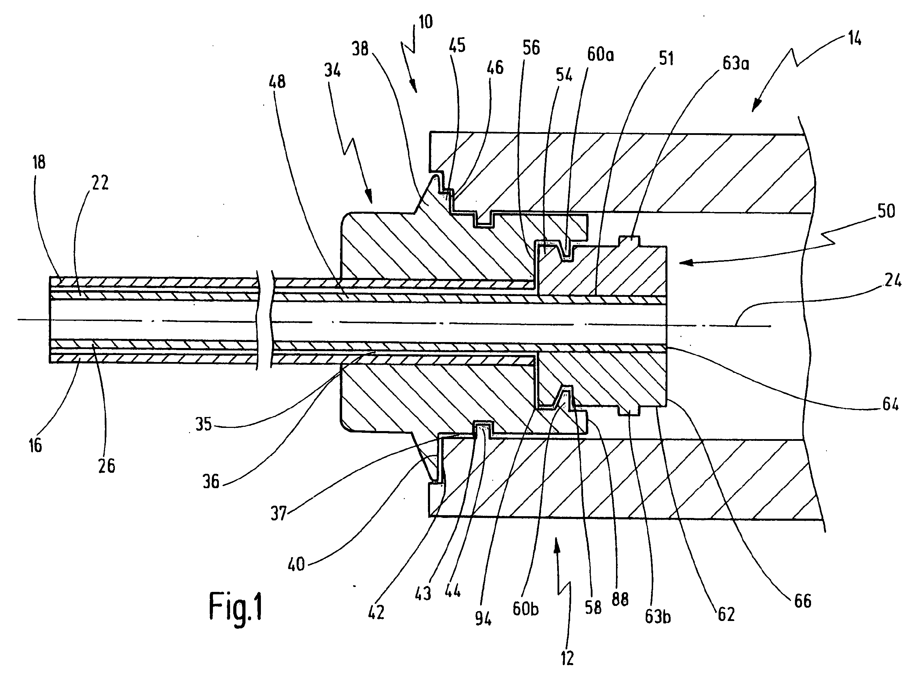

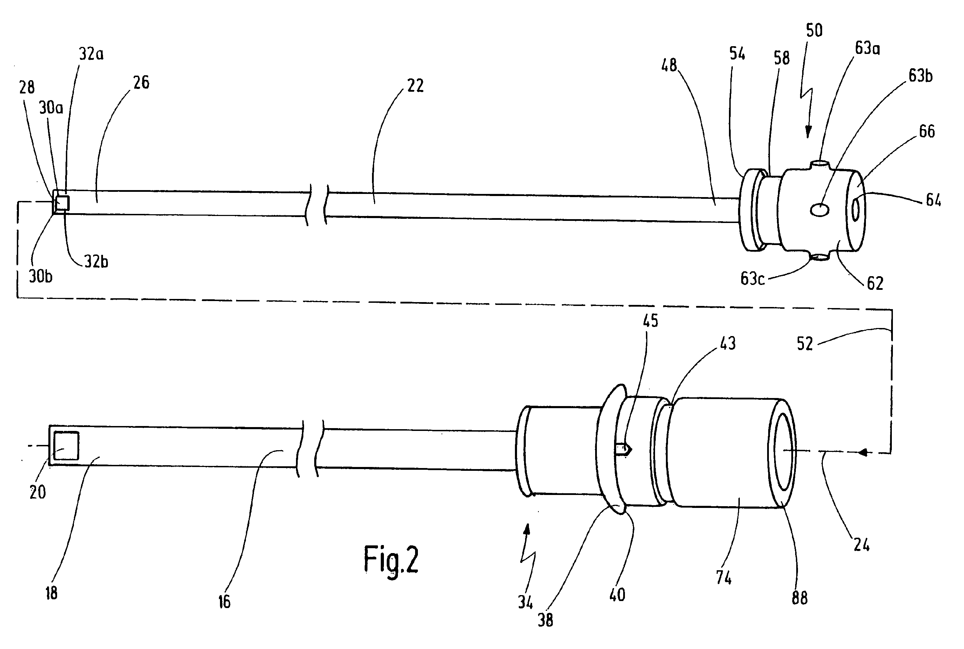

In Fig. 1 ist ein mit dem allgemeinen Bezugszeichen 10 versehenes medizinisches Instrument zum Schneiden von Gewebe dargestellt. Weitere Einzelheiten des medizinischen Instruments 10 sind in Fig. 2 bis 6 dargestellt.

Das medizinische Instrument 10 wird bspw. in der minimal-invasiven Chirurgie zum Abtragen von Gewebe in einem menschlichen oder tierischen Körper verwendet.

Das medizinische Instrument 10 weist an seinem proximalen Ende 12 einen abnehmbaren Handgriff 14 auf, an dem distalseitig ein Außenschaft 16 angeordnet ist. Der Außenschaft 16 ist als starres Hohlrohr ausgebildet und weist an seinem distalen Ende 18 ein halbumfänglich ausgebildetes Fenster 20 auf. In dem Außenschaft 16 ist ein ebenfalls als starres Hohlrohr ausgebildeter Innenschaft 22 aufgenommen, der um eine Längsachse 24 des medizinischen Instruments 10 drehbar ausgebildet ist. Der Innenschaft 22 weist im Bereich seines distalen Endes 26 ein Fenster 28 auf, das axial im Bereich des Fensters 20 des Außenschafts 16 angeordnet ist, so dass die Fenster 20, 28 im aufgenommenen Zustand des Innenschafts 22 etwa auf gleicher axialer Höhe liegen (vgl. Fig. 2). Das Fenster 28 des Innenschafts 22 ist mit zumindest einem Schneidelement 30, hier dargestellt mit zwei Schneidelementen 30a, b, versehen, die beidseitig an parallel zur Längsachse 24 angeordneten Kanten 32a, b des Fensters 28 vorgesehen sind.

Der Innenschaft 22 ist zum Abtragen von seitlich des Außenschafts 16 befindlichem Gewebe in Rotation versetzbar, so dass die Schneidelemente 30a, b am Fenster 20 des Außenschafts 16 vorbei laufen und das Gewebe abtrennen.

Der Außenschaft 16 und der Innenschaft 22 sind aus Edelstahl mit geeigneter Wandstärke ausgebildet, so dass beide Bauteile eine ausreichende Steifigkeit gegen beim Schneidvorgang auftretenden Torsionskräften aufweisen. Ein Durchmesser des Außenschafts liegt im Bereich von wenigen Millimetern und ist folglich derart klein ausgebildet, das der Arzt vor Einführen des distalen Endes 18 des Außenschafts 16 in den Körper nur einen keinen Schnitt in eine äußere Haut eines Patienten ausführen muss.

Der Außenschaft 16 ist drehfest mit einem Kupplungselement 34 verbunden, über das der Außenschaft 16 mit dem Handgriff 14 verbindbar ist. Bei dem in Fig. 1 bis 6 gezeigten medizinischen Instrument 10 ist der Außenschaft 16 mit einer inneren Oberfläche 35 einer axial verlaufenden Bohrung 36 des Kupplungselements 34 verklebt. Das Kupplungselement 34 ist über eine Steckverbindung zumindest teilweise in einer distalseitigen Öffnung 37 des Handgriffs 14 aufnehmbar. Hierzu weist das Kupplungselement 34 eine vollumlaufende ringförmige Verbreiterung 38 auf, deren proximalseitig angeordnete Oberfläche 40 in einer entsprechenden ringförmigen Vertiefung 42 des Handgriffs 14 einschiebbar ist. Das Kupplungselement 34 weist ferner eine vollumfängliche Nut 43 auf, in die eine ringförmige Verbreiterung 44 des Handgriffs 14 eingreift. Hierdurch wird eine axiale Lageverschiebung des Handgriffs 14 und des Kupplungselements 34 verhindert. Um eine drehfeste Verbindung des Kupplungselements 34 mit dem Handgriff 14 herzustellen, weist das Kupplungselement 34 ferner zumindest einen Steg 45 auf, der direkt benachbart zur Oberfläche 40 der ringförmigen Verbreiterung 38 angeordnet und in eine entsprechende Aufnahme 46 des Handgriffs 14 formschlüssig einrastbar ist. Das Kupplungselement 34 kann auch mehrere Stege 45 aufweisen, die in jeweilige Aufnahmen 46 des Handgriffs 14 einschiebbar sind.

Ein proximaler Endbereich 48 des Innenschafts 22 ist drehfest mit einem Rotationsübertragungselement 50 verbunden, indem er mit einer inneren Oberfläche 51 des Rotationsübertragungselements 50 verklebt ist. In einem aufgenommenen Zustand ist der Innenschaft 22 entlang eines in Fig. 2 dargestellten Pfeils 52 in dem Außenschaft 16 eingebracht, so dass eine distale Stirnfläche 54 des Rotationsübertragungselements 50 an einer proximalen Stirnfläche 56 des Kupplungselements 34 anliegt. Das Rotationsübertragungselement 50 weist ferner eine vollumlaufende Nut 58 auf, in die umfänglich angeordnete Steckelemente 60 des Kupplungselements 34, in Fig. 1 dargestellt zwei Steckelemente 60a, b, formschlüssig eingreifen, so dass das Rotationsübertragungselement 50 und das Kupplungselement 34 zueinander axial fest, aber frei drehbar angeordnet sind.

Das Kupplungselement 34 und das Rotationsübertragungselement 50 sind aus einem Kunststoffmaterial, bspw. PVC, gefertigt.

Ferner sind an eine äußere Oberfläche 62 des Rotationsübertragungselements 50 Antriebsmittel (nicht dargestellt), bspw. ein Elektromotor, anbringbar, die eine Rotationsbewegung des Rotationsübertragungselements 50 relativ zum Kupplungselement 34, d. h. des Innenschafts 22 relativ zum Außenschaft 16, bewirken. Hierzu sind an der äußeren Oberfläche 62 des Rotationsübertragungselements 50 ellipsenförmige, im Wesentlichen parallel zur Längsachse 24 angeordnete Angreifelemente 63, in Fig. 1 dargestellt zwei Angreifelemente 63a, b, vorgesehen, an denen eine Antriebswelle der Antriebsmittel angreifen.

Es ist ferner eine Saugleitung (nicht dargestellt) an einem proximalen Ende 64 des Innenschafts 22 anbringbar, die bspw. über eine Steckverbindung in das proximale Ende 64 des Innenschafts 22 eingreift. Das proximale Ende 64 des Innenschafts 22 schließt mit einer nach proximal weisenden Stirnfläche 66 des Rotationsübertragungselements 50 ab, so dass ein distales Ende der Saugleitung direkt an der Stirnfläche 66 des Rotationsübertragungselements 50 angeordnet werden kann und somit eine flüssigkeitsdichte Verbindung des Innenschafts 22 und der Saugleitung erzeugt wird.

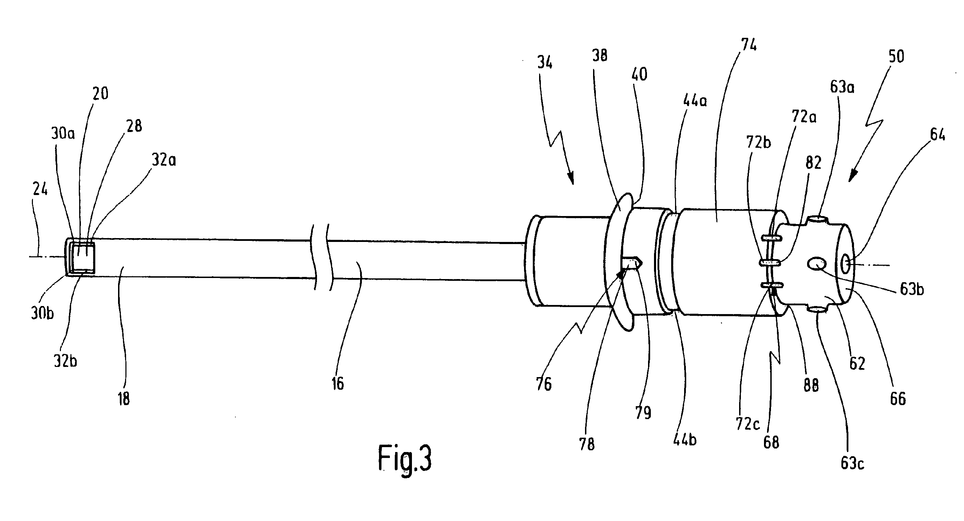

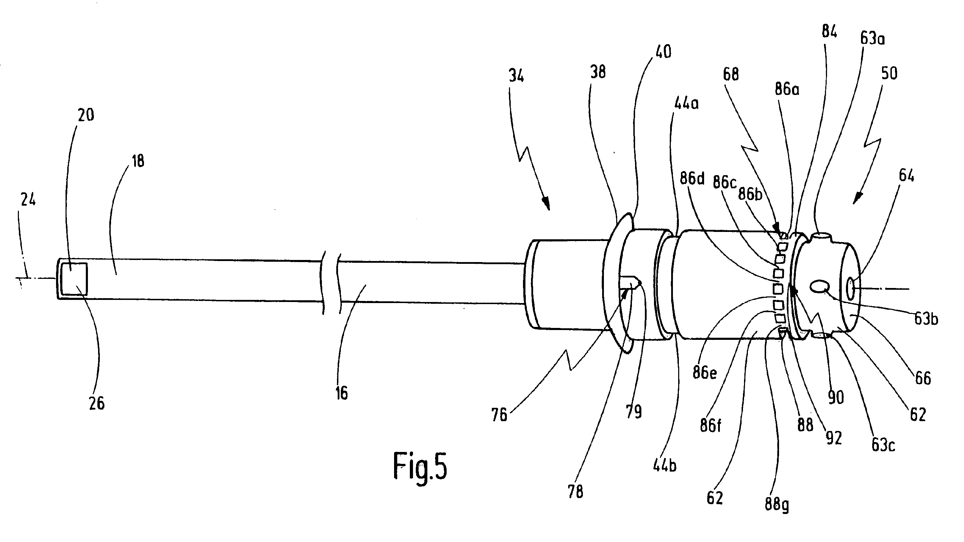

Das medizinische Instrument 10 weist ferner ein durch eine Rotationsbewegung des Innenschafts 22 irreversibel lösbares Fixierelement 68 auf, das in seinem ungelösten Zustand den Innenschaft 22 relativ zum Außenschaft 16 drehfest in einer vorgegebenen Stellung hält. Der Außenschaft 16 und der Innenschaft 22 können bspw. derart angeordnet sein, dass das Fenster 28 des Innenschafts 22 dem Fenster 20 des Außenschafts 16 entgegengesetzt angeordnet ist, so dass das Fenster 20 des Außenschafts 16 durch das distale Ende 26 des Innenschafts 22 verschlossen ist (vgl. Fig. 5). Diese Anordnung der Fenster 20, 28 ist insbesondere im Rahmen von Eingriffen vorteilhaft, bei denen das distale Ende 18 des Außenschafts 16 in eine Körperöffnung eingeführt wird, während die Saugleitung an das Rotationsübertragungselement 50 angeschlossen und aktiviert ist. Der Innenschaft 22 kann ebenfalls bezüglich des Außenschafts 16 derart angeordnet sein, dass das Fenster 20 des Außenschafts 16 maximal geöffnet ist (vgl. Fig. 3). Diese Stellung beider Fenster 20, 28 kann für operative Eingriffe im Bereich der Hals-Nasen-Ohren-Heilkunde bevorzugt sein. Hierbei weisen das Fenster 20 des Außenschafts 16 und das Fenster 28 des Innenschafts 22 in eine gleiche Richtung, so dass auch beim Einführen des distalen Endes 18 des Außenschafts 16 Flüssigkeit oder Gewebe aus dem Operationsgebiet abgesaugt werden kann. Es ist ebenfalls möglich, dass der Innenschaft 22 relativ zum Außenschaft 16 in jeder möglichen Stellung drehfest gehalten wird.

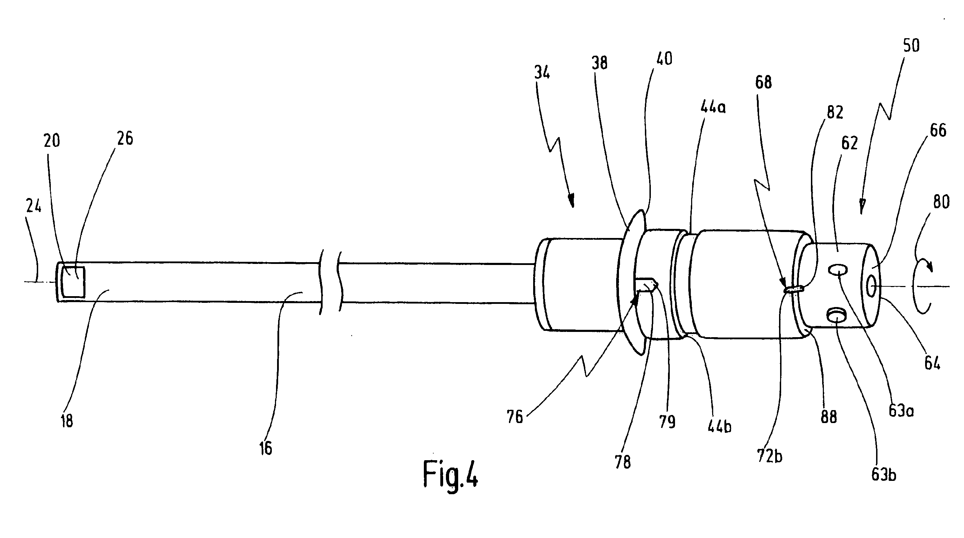

In einem ersten, in Fig. 3, 4 dargestellten Ausführungsbeispiel weist das Fixierelement 68 zumindest einen länglichen Steg 72 aus Kunststoff, hier drei längliche Stege 72a-c, auf, die eine äußere Oberfläche 74 des Kupplungselements 34 mit der äußeren Oberfläche 62 des Rotationsübertragungselements 50 verbinden. Die Kunststoffstege 72a-c sind entlang eines Umfangs des Kupplungselements 34 und des Rotationsübertragungselements 50 in regelmäßigen Abständen, bspw. jeweils unter 45° zur Längsachse 24, angeordnet.

Das Kupplungselement 34, die Kunststoffstege 72a-c und das Rotationsübertragungselement 50 sind bei ihrer Fertigung durch Spritzgießen einstückig ausgebildet, so dass der Innenschaft 22 relativ zum Außenschaft 16 in der jeweils fertigungsbedingten vorgegebenen Stellung angeordnet ist. Die drei Bauteile können auch mittels Extrusionstechnik gefertigt sein.

Um die Relativstellung des Außen- und Innenschafts 16, 22 zueinander auszurichten und nachträglich überprüfen zu können, weist das medizinische Instrument 10 ferner einen Positionsindikator 76 auf, der in Form eines nach proximal weisenden Pfeils 78 auf der Oberfläche 74 des Kupplungselements 34 direkt benachbart zur ringförmigen Verbreiterung 38 angeordnet ist. In den in Fig. 3-6 dargestelltem Ausführungsbeispiel des Positionsindikators 76 entspricht dieser dem Steg 45. Bei der Herstellung des medizinischen Instruments 10 wird darauf geachtet, dass der Kunststoffsteg 72b in Flucht mit bspw. einer Spitze 79 des Pfeils 78 angeordnet ist, so dass, wie in Fig. 3 dargestellt, das Fenster 20 des Außenschafts 16 geöffnet ist. Hierbei kann der Kunststoffsteg 72b zusätzlich farblich besonders gekennzeichnet sein und/oder es kann zusätzlich eine ebenfalls in Flucht mit dem Positionsindikator 76 angeordnete Markierung auf der äußeren Oberfläche 62 des Rotationsübertragungselements 50 vorgesehen sein, so dass die relative Anordnung des Außen- und Innenschafts 16, 22 einfach eingestellt und überprüft werden kann.

Der Positionsindikator 76 kann ebenfalls auf einem Bereich der äußeren Oberfläche 62 des Rotationsübertragungselements 50 aufgebracht sein, der bspw. dann sichtbar ist, wenn das Kupplungselement 34 noch nicht im Handgriff 14 aufgenommen ist. Der Positionsindikator 76 kann anstelle des nach proximal weisenden Pfeils 78 als nach distal weisender Pfeil oder als kleiner, farblich zum Kupplungselement 34 und Rotationsübertragungselement 50 unterscheidbarer Punkt ausgebildet sein.

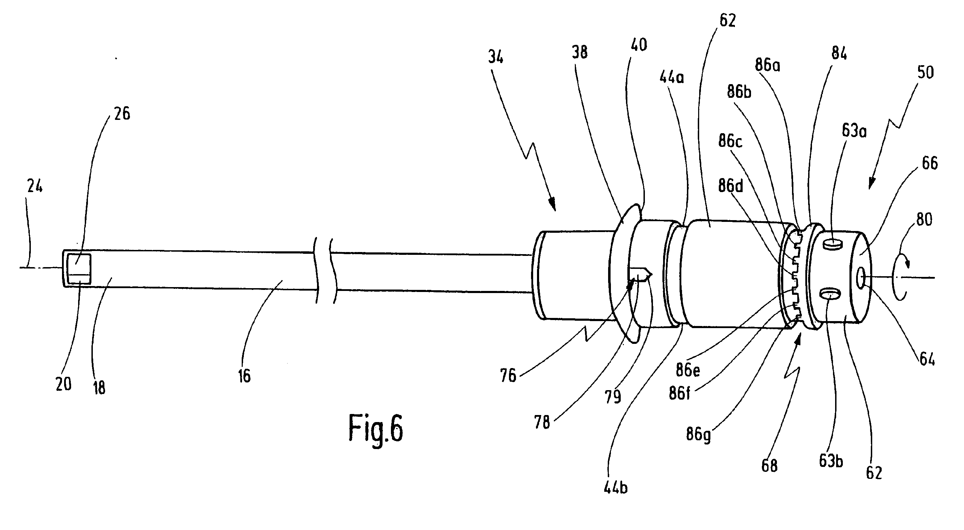

Wenn die Schneidelemente 30a, b bspw. nach Einführen des distalen Endes 18 des Außenschafts 16 in die Körperöffnung in Betrieb genommen wird, verursachen die Antriebsmittel eine Rotationsbewegung des Rotationsübertragungselement 50 in Richtung eines in Fig. 4 und 6 dargestellten Pfeils 80, die auf den Innenschaft 22 übertragen wird. Der Außenschaft 16 verleibt in seiner Stellung und rotiert nicht um die Längsachse 24. Es ist ebenfalls möglich, dass der Innenschaft 22 entgegengesetzt zu der durch den Pfeil 80 angedeuteten Rotationsrichtung rotiert.

Die Rotationsbewegung des Innenschafts 22 erzeugt Torsions- und Scherkräfte auf die Kunststoffstege 72a-c, die bei genügend großer Krafteinwirkung an einer Sollbruchstelle zerbrechen, wodurch die drehfeste Anordnung des Außen- und Innenschafts 16, 22 irreversibel gelöst ist. Wie in Fig. 4 dargestellt, ist der Kunststoffsteg 72b an seinem proximalen Ende 82 von der Oberfläche 62 des Rotationsübertragungselements 50 abgebrochen, so dass er nur noch an der äußeren Oberfläche 74 des Kupplungselements 34 befestigt ist.

Die Stege 72a-c können ferner bspw. in ihrem Mittelbereich eine Perforierung aufweisen, um ihr Brechen in diesem definierten Bereich zu unterstützen. Diese Perforierung wirkt hierbei als Sollbruchstelle des Fixierelements 68.

Das medizinische Instrument 10 ist aufgrund des irreversibel lösbaren Fixierelements 68 insbesondere als Einweginstrument geeignet.

In einem in Fig. 5 dargestellten, weiteren Ausführungsbeispiel ist das Fixierelement 68 als vollumfänglicher Kunststoffring 84 ausgebildet, der über kleine Kunststoffbrücken 86, hier dargestellt sieben Kunststoffbrücken 86a-g, mit einer proximalseitigen Stirnfläche 88 des Kupplungselements 34 verbunden ist. Der Kunststoffring 84, die Kunststoffbrücken 86a-g und das Kupplungselement 34 sind einstückig spritzgegossen.

Der Kunststoffring 84 sitzt reibschlüssig auf der äußeren Oberfläche 62 des Rotationsübertragungselements 50 auf, so dass das Rotationsübertragungselement 50 und das Kupplungselement 34 in einem Ruhezustand drehfest zueinander angeordnet sind. Hierdurch ist der Innenschaft 22 und der Außenschaft 16 in einer solchen Stellung angeordnet, bei der, wie in Fig. 5 dargestellt, das distale Ende 26 des Innenschafts 22 das Fenster 20 des Außenschafts 16 verschließt. Der Positionsindikator 76 ist in Flucht mit einem durch seine Farbgebung besonders markierten Bereich 90 des Kunststoffrings 84, hier dargestellt mit einem farbigen Punkt 92, angeordnet.

Wie in Fig. 6 dargestellt, bewirkt die Rotationsbewegung des Innenschafts 22 entlang des Pfeils 80 eine Verdrehung des Rotationsübertragungselements 50 relativ zum Kupplungselement 34, so dass Torsions- und Scherkräfte an den Kunststoffbrücken 86a-g angreifen. Der Ring 84 bricht unter dieser Krafteinwirkung entlang der Kunststoffbrücken 86a-g vom Kupplungselement 34 ab, die eine Sollbruchlinie des Fixierelements 68 definieren. Der vom Kupplungselement 34 abgetrennte Kunststoffring 84 verbleibt während der restlichen Zeit des operativen Eingriffs in dieser Position.

Es ist ebenfalls möglich, dass das Fixierelement 68 aus biegeflexiblen, reißfähigen Material, bspw. Gummi, ausgebildet ist, das zwischen der äußeren Oberfläche 62 des Rotationsübertragungselements 50 und einer inneren Oberfläche 94 des Kupplungselements 34 angeordnet ist. Durch ein bei der Rotationsbewegung auftretendes Drehmoment reißt das Gummi und löst den Innenschaft 22 irreversibel vom Außenschaft 16.

## Patentansprüche

1. Medizinisches Instrument zum Schneiden von Gewebe, mit einem rohrförmigen Außenschaft (16), der im Bereich seines distalen Endes (18) zumindest ein Fenster (20) aufweist, mit einem rohrförmigen, um eine Längsachse (24) rotierbaren, in dem Außenschaft (16) aufgenommenen Innenschaft (22), der an seinem distalen Ende (26) ein im Bereich des zumindest einen Fensters (20) des Außenschafts (16) angeordnetes und mit zumindest einem Schneidelement (30a, b) versehenes Fenster (28) aufweist, **gekennzeichnet durch** ein Fixierelement (68) zum drehfesten Halten des Innenschafts (22) relativ zum Auβenschaft (16) in einer vorgegebenen Stellung, wobei das Fixierelement (68) durch eine Rotationsbewegung des Innenschafts (22) irreversibel lösbar ist.

2. Medizinisches Instrument nach Anspruch 1, **dadurch gekennzeichnet, dass** das Fixierelement (68) ein mit dem Außenschaft (16) drehfest verbundenes Kupplungselement (34) zum Verbinden des Außenschafts (16) mit einem Handgriff (14) mit einem mit dem Innenschaft (22) drehfest verbundenen Rotationsübertragungselement (50) zum Verbinden des Innenschafts (22) mit Antriebsmitteln verbindet.

3. Medizinisches Instrument nach Anspruch 2, **dadurch gekennzeichnet, dass** das Fixierelement (68) an einer äußeren Oberfläche (74, 62) des Kupplungselements (34) und des Rotationsübertragungselements (50) angeordnet ist.

4. Medizinisches Instrument nach Anspruch 2 oder 3, **dadurch gekennzeichnet, dass** das Fixierelement (68) zumindest teilweise umfänglich am Kupplungselement (34) und/oder Rotationsübertragungselement (50) angeordnet ist.

5. Medizinisches Instrument nach einem der Ansprüche 2 bis 4, **dadurch gekennzeichnet, dass** das Kupplungselement (34) und das Fixierelement (68) einstückig ausgebildet sind.

6. Medizinisches Instrument nach einem der Ansprüche 2 bis 5, **dadurch gekennzeichnet, dass** das Rotationsübertragungselement (50) und das Fixierelement (68) einstückig ausgebildet sind.

7. Medizinisches Instrument nach einem der Ansprüche 1 bis 6, **dadurch gekennzeichnet, dass** das Fixierelement (68) zumindest einen länglichen Steg (72a-c) aufweist, der durch die Rotationsbewegung des Innenschafts (22) an zumindest einer Sollbruchstelle bricht.

8. Medizinisches Instrument nach Anspruch 7, **dadurch gekennzeichnet, dass** der zumindest eine Steg (72a-c) entlang der Längsachse (24) angeordnet ist.

9. Medizinisches Instrument nach einem der Ansprüche 1 bis 6, **dadurch gekennzeichnet, dass** das Fixierelement (68) einen Ring (84) aufweist, der durch die Rotationsbewegung des Innenschafts (22) entlang einer Sollbruchlinie bricht.

10. Medizinisches Instrument nach einem der Anspruch 1 bis 9, **gekennzeichnet durch** einen Positionsindikator (76) zum Ausrichten des Innenschafts (22) relativ zum Außenschaft (16) in der vorgegebenen Stellung, wobei der Positionsindikator (76) in einer Flucht mit dem Fixierelement (68) angeordnet ist.

11. Medizinisches Instrument nach Anspruch 10, **dadurch gekennzeichnet, dass** der Positionsindikator (76) an der äußeren Oberfläche (74) des Kupplungselements (34) angeordnet ist.

## Claims

1. A medical instrument for cutting tissue, comprising a tubular outer shaft (16), which has at least one window (20) in the region of its distal end (18), further comprising a tubular inner shaft (22) which is rotatable about a longitudinal axis (24) and housed in the outer shaft (16) and which has at its distal end (26) a window (28) arranged in the region of the at least one window (20) of the outer shaft (16) and provided with at least one cutting element (30a, b), **characterized by** a fixing element (68) for torque-proof holding of the inner shaft (22) relative to the outer shaft (16) in a preset position, the fixing element (68) being irreversibly detachable through rotational movement of the inner shaft (22).

2. The medical instrument of claim 1, **characterized in that** the fixing element (68) connects a coupling element (34) connected torque-proof to the outer shaft (16) for connecting the outer shaft (16) to a handgrip (14) with a rotation transfer element (50) connected torque-proof to the inner shaft (22) for connecting the inner shaft (22) to drive means.

3. The medical instrument of claim 1 or 2, **characterized in that** the fixing element (68) is arranged on an external surface (74, 62) of the coupling element (34) and of the rotation transfer element (50).

4. The medical instrument of any one of claims 1 through 3, **characterized in that** the fixing element (68) is arranged at least partially peripherally on the coupling element (34) and/or rotation transfer element (50).

5. The medical instrument of any one of claims 1 through 4, **characterized in that** the coupling element (34) and the fixing element (68) are designed in one piece.

6. The medical instrument of any one of claims 1 through 5, **characterized in that** the rotation transfer element (50) and the fixing element (68) are designed in one piece.

7. The medical instrument of any one of claims 1 through 6, **characterized in that** the fixing element (68) has at least one oblong web (72a-c) which breaks, due to the rotational movement of the inner shaft (22), at at least one nominal breaking point.

8. The medical instrument of claim 7, **characterized in that** the at least one web (72a-c) is arranged along the longitudinal axis (24).

9. The medical instrument of any one of claims 1 through 6, **characterized in that** the fixing element (68) has a ring (84), which breaks along a nominal breaking line due to the rotational movement of the inner shaft (22).

10. The medical instrument of any one of claims 1 through 9, **characterized by** a position indicator (76) for aligning the inner shaft (22) relative to the outer shaft (16) in the preset position, the position indicator (76) being arranged in alignment with the fixing element (68).

11. The medical instrument of claim 10, **characterized in that** the position indicator (76) is arranged on the external surface (74) of the coupling element (34).

## Revendications

1. Instrument médical destiné à couper des tissus, avec une tige extérieure (16) de forme tubulaire, laquelle présente dans la zone de son extrémité distale (18) au moins une fenêtre (20), avec une tige intérieure (22) de forme tubulaire logée dans la tige extérieure (16), pouvant tourner autour d'un axe longitudinal (24), qui présente à son extrémité distale (26) une fenêtre (28) disposée dans la zone d'au moins une fenêtre (20) de la tige extérieure (16) et munie d'au moins un élément de découpe (30a, b), **caractérisé par** un élément de fixation (68) pour le maintien résistant à la rotation de la tige intérieure (22) par rapport à la tige extérieure (16) dans une position prescrite, l'élément de fixation (68) pouvant être enlevé de façon irréversible grâce un mouvement de rotation de la tige intérieure (22).

2. Instrument médical selon la revendication 1, **caractérisé en ce que** l'élément de fixation (68) relie un élément d'accouplement (34) relié de manière résistante à la rotation à la tige extérieure (16) pour relier la tige extérieure (16) à une poignée (14), à un élément transmetteur de rotation (50) relié de manière résistante à la rotation à la tige intérieure (22) pour relier la tige intérieure (22) à des moyens d'entraînement.

3. Instrument médical selon la revendication 2, **caractérisé en ce que** l'élément de fixation (68) est disposé contre une surface extérieure (74, 62) de l'élément d'accouplement (34) et de l'élément transmetteur de rotation (50).

4. Instrument médical selon la revendication 2 ou 3, **caractérisé en ce que** l'élément de fixation (68) est disposé au moins partiellement sur le pourtour de l'élément d'accouplement (34) et/ou de l'élément transmetteur de rotation (50).

5. Instrument médical selon l'une quelconque des revendications 2 à 4, **caractérisé en ce que** l'élément d'accouplement (34) et l'élément de fixation (68) sont conçus de manière monobloc.

6. Instrument médical selon l'une quelconque des revendications 2 à 5, **caractérisé en ce que** l'élément transmetteur de rotation (50) et l'élément de fixation (68) sont conçus de manière monobloc.

7. Instrument médical selon l'une quelconque des revendications 1 à 6, **caractérisé en ce que** l'élément de fixation (68) présente au moins une entretoise longitudinale (72 a-c), qui se rompt grâce au mouvement de rotation de la tige intérieure (22) à au moins un point de rupture prescrit.

8. Instrument médical selon la revendication 7, **caractérisé en ce qu'**au moins une entretoise (72 a-c) est disposée le long de l'axe longitudinal (24).

9. Instrument médical selon l'une quelconque des revendications 1 à 6, **caractérisé en ce que** l'élément de fixation (68) présente un anneau (84), qui se rompt grâce au mouvement de rotation de la tige intérieure (22) le long d'une ligne de rupture prescrite.

10. Instrument médical selon l'une quelconque des revendications 1 à 9, **caractérisé par** un indicateur de position (76) pour l'ajustement de la tige intérieure (22) par rapport à la tige extérieure (16) dans la position prescrite, l'indicateur de position (76) étant disposé en alignement avec l'élément de fixation (68).

11. Instrument médical selon la revendication 10, **caractérisé en ce que** l'indicateur de position (76) est disposé contre la surface extérieure (74) de l'élément d'accouplement (34).
